# EUROPEAN PATENT APPLICATION

(11) **EP 4 520 331 A1**
(43) Date of publication of application: **12.03.2025**
(21) Application number: 23196201.0
(22) Date of filing: 08.09.2023
(51) Int. Cl.: A61K 31/4709, A61K 31/713, A61P 43/00

(54) **PHARMACEUTICAL COMPOSITION COMPRISING TRPV4 INHIBITOR FOR USE IN THE PREVENTION OR TREATMENT OF CEREBRAL CAVERNOUS MALFORMATIONS (CCM)**

(71) Applicant: Université Grenoble Alpes, 38400 Saint-Martin-d'Hères (FR); Institut National de la Santé et de la Recherche Médicale, 75013 Paris (FR); Centre National de la Recherche Scientifique, 75016 Paris (FR); Université de Poitiers, 86034 Poitiers Cedex (FR); Katholieke Universiteit Leuven, 3000 Leuven (BE); INOVOTION, 38700 La Tronche (FR)
(72) Inventor: FAUROBERT, Eva, 38700 Le Sappey-en-Chartreuse (FR); PASQUIER, Candice, 38100 Grenoble (FR); DESTAING, Olivier, 38100 Grenoble (FR); PENNA, Aubin, 86280 Saint-Benoit (FR); VAN OOSTERWYCK, Hans, 3010 Leuven (BE); DE JONG, Janne, 2550 Kontich (BE)
(74) Representative: Germain Maureau

(57) **Abstract**

The present invention concerns a pharmaceutical composition for use in the prevention or treatment of cerebral cavernous malformations (CCM) comprising a transient receptor potential vanilloid 4 (TRPV4) inhibitor.

## Description

### FIELD OF THE INVENTION

The present invention relates to the field of therapeutic agent for the prevention or treatment of cerebral cavernous malformations (CCM).

### STATE OF THE ART

Cerebral Cavernous Malformations (CCM) occur in 0.5% of the world's population. They account for a large proportion (8-15%) of all cerebral and spinal vascular malformations. People with familial CCM (20% of cases) generally develop multiple lesions that progress over time. Familial CCM are associated with mutations in either one of specific genes, among the latest, but not limited to them, are the gene KRIT1 aso called CCM1, the gene MGC4607 also called CCM2 , and the gene PDCD10 also called CCM3.

The most frequent clinical manifestations are epilepsy, migraines, hemorrhages and numerous other associated neurological deficits such as paralysis of the arms or legs, hearing or visual impairments, or cognitive deficits such as difficulties with speech, memory and attention.

CCM (Cerebral Cavernous Malformations) lesions are formed by stacks of tortuous, dilated and hemorrhagic capillaries located in the brain. These brain capillaries are devoid of mural cells and are formed of a monolayer of weakly joined endothelial cells (EC). 3D reconstruction of human CCM lesions has demonstrated the presence of a profusion of endothelial buds dilated into caverns that extends into the brain parenchyma from the innervating vessel. These cavernous growths only form in venous capillaries with low blood flow, for a reason that remains unknown.

The major steps of the development of the CCM disease are known to be 1) dedifferentiation/senescence, 2) loss of blood-brain barrier (BBB) (which results in disruption of cell-cell junctions/actin stress fibers and in increasing permeability of the BBB), 3) extracellular matrix (ECM) degradation by secreted matrix metalloproteinases (MMPs) and 4) invasion of the surrounding tissue.

Over the last twenty years, studies on CCM signaling have multiplied with the identification of several pathways deregulated downstream of CCM gene mutations. The most extensively studied are RhoA/ROCK, VEGF, MEKK3-KLF2/4, PI3K/AKT/mTOR and reactive oxygen species.

Drugs targeting RhoA/ROCK-related signaling pathways (ROCK inhibitors and statins) have been among the most promising. However, the undesirable side effects of these treatments have prompted the search for other more specific targets.

At the present time, there is no proven and safe treatment for CCM and surgical removal is not always possible, depending on the location of the lesion(s) in the brain.

There is, therefore, a real need to understand the pathophysiological mechanisms involved in the formation of these vascular malformations and thus identify therapeutic targets.

Transient receptor potential vanilloid 4 (TRPV4), a member of the TRP superfamily, is a channel permeable to Ca2+. In the central nervous system, TRPV4 is expressed in various cell types, including neurons, glial cells and vascular endothelial cells. TRPV4 is known to play a pivotal role in central nervous system disorders, such as ischemic stroke, traumatic brain injury and Alzheimer's disease. TRPV4 is also known for its restorative effect on Blood-brain barrier (BBB) in a model of intracerebral hemorrhage (H. Zhao et al., TRPV4 Blockade Preserves the Blood-Brain Barrier by Inhibiting Stress Fiber Formation in a Rat Model of Intracerebral Hemorrhage Front. Mol. Neurosci. 11 (2018), doi:10.3389/fnmol.2018.00097).

### DESCRIPTION OF THE INVENTION

The inventors have surprisingly identified in their research that the transient receptor potential vanilloid 4 (TRPV4) had an impact upstream of the deregulated pathways in cells with CCM gene deficiencies. More precisely, inventors had identified that TRPV4 was exceptionally highly over-expressed, at the mRNA level as well as at the protein level in these endothelial cells, and is involved in many steps of the CCM disease, including steps 1) Dedifferentiation/Senescence, 2) Loss of Blood-brain barrier (BBB) and 3) Extracellular matrix (ECM) degradation by secreted Matrix metalloproteinases (MMPs).

Accordingly, the present invention relates to a pharmaceutical composition for use in the prevention or treatment of cerebral cavernous malformations (CCM), comprising a transient receptor potential vanilloid 4 (TRPV4) inhibitor.

The inventors have discovered that the use of one or more TRPV4 inhibitors, or of a pharmaceutical composition comprising such a TRPV4 inhibitor, in endothelial cells with CCM gene deficiencies, has a restorative effect on said first steps of the CCM disease. The use of a pharmaceutical composition according to the invention can block or delay the dedifferentiation of the cell, reverse permeability of BBB and block or reduce ECM degradation. In addition, TRPV4 channels have the advantage of being accessible directly from the bloodstream, thus bypassing the problem of penetrating through the BBB. In this way, TRPV4 inhibitors (notably chemical compounds) can reach TRPV4 channels directly without having to be transported across the BBB or without the risk of being damaged or blocked by the BBB.

In the context of the present invention:
- "treatment" designates a method or process that is aimed at (1) delaying or preventing the onset of a disease or condition (here CCM disease); (2) slowing down or stopping the progression, aggravation, or deterioration of the symptoms of the disease or condition; (3) bringing about amelioration of the symptoms of the disease or condition; or (4) curing the disease or condition. A treatment may be administered after initiation of the disease or condition, for a therapeutic action. Alternatively, a treatment may be administered prior to the onset of the disease or condition, for a prophylactic or preventive action. In this case, the term "prevention" is used.
- "transient receptor potential vanilloid 4 (TRPV4) inhibitor", "TRPV4 inhibitor", "transient receptor potential vanilloid 4 (TRPV4) blocker", "TRPV4 blocker" "transient receptor potential vanilloid 4 (TRPV4) repressor" or "TRPV4 repressor" are used interchangeably and designate any therapeutic agent suitable for use in the therapeutic applications of the present invention and which allows to reduce, or even completely inhibited/suppressed, the expression of a gene or of a protein which are normally expressed in the cells. TRPV4 inhibitors comprise therapeutic agents which may block the activity of TRPV4, inhibit the expression of TRPV4, destroy, degrade or cleave TRPV4 mRNAs or protein, block TRPV4 mRNA translation, or target a therapeutic moiety to TRPV4-overexpressing cells.
- "TRPV4 RNA interfering agent" and "TRPV4 RNAi agent" are used interchangeably and designate any RNA molecule that is capable of specifically inhibiting or down-regulating the expression of a target gene (here the TRPV4 gene). By "silencing, inhibiting or down- regulating expression of a target gene", it is meant that the expression of the target gene, or level of RNA molecules or equivalent RNA molecules encoding one or more proteins or protein subunits, or activity of one or more proteins or protein subunits, is reduced below that observed in the absence of the RNAi agent.

- "RNA interference" (or "RNAi") designates any biological process in which RNA molecules silence, inhibit or down-regulate gene expression by causing the destruction, degradation and/or cleavage of specific mRNA molecules or by blocking the translation thereof. As known in the art, RNA interference is now exploited in therapy. Indeed, RNAi can be initiated by the hand of man, for example, to silence the expression of target genes.
- "TRPV4 siRNA" or "TRPV4 small interfering RNA" are used herein interchangeably and designate any RNA (or RNA analog) comprising between about 10-50 nucleotides (or nucleotide analogs) which is capable of directing or mediating TRPV4 RNAi. Preferably, a siRNA comprises between about 15-30 nucleotides or nucleotide analogs, more preferably between about 16-25 nucleotides (or nucleotide analogs), even more preferably between about 18-23 nucleotides (or nucleotide analogs), and even more preferably between about 19-22 nucleotides (or nucleotide analogs) (e.g., 19, 20, 21 or 22 nucleotides or nucleotide analogs).
- "TRPV4 aiRNA" or "TRPV4 asymmetric interfering RNA" are used herein interchangeably and designate any TRPV4 siRNA which is characterized by the length asymmetric between the two RNA strands.
- "TRPV4 shRNA" or "TRPV4 short hairpin RNA" are used herein interchangeably and designate a sequence of RNA having one or more loop structures and a stem comprising self-complementary sense and antisense regions, wherein the antisense region comprises a sequence complementary to a region of the target TRPV4 mRNA. A short hairpin RNA is cleaved by the cellular machinery into siRNA. The stem can be 29, 28, 27, 26, 25, 24, 23, 22, 21, 20, 19, 18, 17, 16, 15, 14, 13, 12, 11 or 10 base pairs in length, for example between 19 and 25 base pairs, or between 19 and 21 base pairs in length. The loop can vary in length. For example, the loop may be 5, 6, 7, 8, 9, 10, 11, 12 or 13 nucleotides in length. The hairpin structure can also contain 3' or 5' overhang portions. For example, the overhang is a 3' or a 5' overhang 1, 2, 3, 4 or 5 nucleotides in length.
- "micro-RNA" or "miRNA" are used herein interchangeably and designate a major group of noncoding RNAs that are known to regulate almost a third of all the coding genes. They are small (~ 20-25 nucleotides long) endogenously formed repressors of gene expression. miRNAs usually bind to the 3' untranslated region (3 'UTR) of the target RNA transcripts (mRNAs or circRNAs) and are capable of inducing posttranscriptional gene regulation by blocking translation or by degrading the target RNAs, or by doing both. miRNAs can also be chemically synthesized. In contrast to siRNA, which has perfect complementarity to the target RNA transcripts, miRNA binds imperfectly to the target RNA transcripts;
- "TRPV4 antagonists" designate any synthetic or natural molecules which has been described as having TRPV4 blocking activity or properties;
- GSK2193874 designates 3-([1,4'-Bipiperidin]-1'-ylmethyl)-7-bromo-N-(1-phenylcyclopropyl)-2-[3-(trifluoromethyl)phenyl]-4-quinolinecarboxamide and its pharmaceutically acceptable salts;
- GSK2798745 designates 1-(((5S,7S)-3-(5-(2-Hydroxypropan-2-yl)pyrazin-2-yl)-7-methyl-2-oxo-1-oxa-3-azaspiro[4.5]decan-7-yl)methyl)-1H-benzo[d]imidazole-6-carbonitrile and its pharmaceutically acceptable salts;
- HC 067047 designates 2-Methyl-1-[3-(4-morpholinyl)propyl]-5-phenyl-N-[3-(trifluoromethyl)phenyl]-1H-pyrrole-3-carboxamide and its pharmaceutically acceptable salts;
- RN-1734 designates 2,4-Dichloro-N-isopropyl-N-(2-isopropylaminoethyl)benzenesulfonamide and its pharmaceutically acceptable salts;
- GSK3395879 designates 2-[(3R,4S)-4-(4-cyano-3-fluorophenoxy)-3-hydroxy-3-(hydroxymethyl)pyrrolidin-1-yl]sulfonyl-5-(trifluoromethyl)benzonitrile and its pharmaceutically acceptable salts;
- GSK3491943 designates 3-chloro-4-(((3R,4S)-4-(4-chlorophenoxy)-3-hydroxy-3-(hydroxymethyl)pyrrolidin-1-yl)sulfonyl)benzonitrile and its pharmaceutically acceptable salts;
- GSK3527497 designates 4-[(3S,4S)-4-(aminomethyl)-1-(5-chloropyridin-2-yl)sulfonyl-4-hydroxypyrrolidin-3-yl]oxy-2-fluorobenzonitrile and its pharmaceutically acceptable salts;
- RN-9893 designates 2-nitro-N-[4-(4-propan-2-ylpiperazin-1-yl)sulfonylphenyl]-4-(trifluoromethyl)benzamide and its pharmaceutically acceptable salts;
- PF-05214030 designates 4-((1-((2,4-Dichlorophenyl)sulfonyl)-3-hydroxyazetidin-3-yl)methoxy)-2-fluorobenzonitrile and its pharmaceutically acceptable salts;
- "continuous schedule" designates a continuous therapeutic treatment of a patient including the successive administration of one or several therapeutic compositions (whereof multi-therapies according to the invention or not), identical or different, each with its own therapeutic scheduling (number of daily administrations and number of days of administration over a given period, week for example) and thus, in an unlimited manner and unsequenced or spaced apart over time, that is to say, without interruption of the treatment;
- "daily administration" designates an administration once a day or once every 24 hours;
- "pharmaceutically acceptable salts" of an active ingredient designates any salt of addition of said active ingredient with an inorganic or organic acid by the action of such an acid within an organic or aqueous solvent, such as an alcohol, a ketone, an ether or a chlorinated solvent, and which is acceptable from a pharmaceutical point of view; and
- unless otherwise specified, all % values are weight %.

The present invention thus relates to a pharmaceutical composition for use in the prevention or treatment of cerebral cavernous malformations (CCM), comprising a transient receptor potential vanilloid 4 (TRPV4) inhibitor. Preferably, the present invention relates to a pharmaceutical composition as defined above in which TRPV4 inhibitor is chosen as being TRPV4 antagonist such as GSK2193874, GSK2798745, HC 067047, RN-1734, GSK3395879, GSK3491943, GSK3527497, RN-9893, PF-05214030 or TRPV4 RNA interfering agent such as TRPV4 siRNA, TRPV4 shRNA, micro-RNA, aiRNA. More preferably, TRPV4 inhibitor is chosen as being TRPV4 siRNA. Still more preferably, TRPV4 inhibitor is chosen as being GSK2193874, GSK2798745, HC 067047, RN-1734, GSK3395879, GSK3491943, GSK3527497, RN-9893, PF-05214030. Even more preferably, TRPV4 antagonist is chosen as being GSK2193874.The pharmaceutical composition according to the present invention contains the active ingredients in sufficient quantities to ensure the required therapeutic effect on endothelial cells of patients with CCM.

Preferably, the pharmaceutical composition according to the present invention contains from 0.05% to 12% of TRPV4 inhibitor, preferably from 0.1% to 6% of TRPV4 inhibitor.

The pharmaceutical composition according to the present invention can be formulated in any galenic formulation necessary for its administration. Particularly, for topical administration, the composition according to the present invention can be formulated as a gel or a thermogel. For oral administration, the compositions according to the present invention can be formulated in the form of coated or non-coated, effervescent, soluble, orodispersible, gastro-resistant or modified release tablets; sugar-coated pills; hard-shelled capsules (or gelcaps); soft-shelled capsules; granules; pellets; pills; lozenges. For nasal route administration, the composition can be formulated in the form of spray or powder to be inhaled. For systemic route administration, the composition according to the present invention can be formulated in the form of a sterile lyophilized powder for injection.

The pharmaceutical compositions according to the present invention could hence comprise, in addition to the active ingredients, any adjuvant of pharmaceutically acceptable formulation known by the skilled person and essential to the preparation of the pharmaceutical composition in the required form.

The pharmaceutical composition according to the invention can be administered to a patient under therapy for treating CCM between one and four times daily maximum.

The pharmaceutical composition according to the invention can be administered at any time of day, before, during or after therapy for treating CCM without it affecting the effectiveness of the treatment.

The pharmaceutical composition according to the present invention can be administered to the patient once or several times per week. Thus, the present invention also relates to a pharmaceutical composition such as previously defined to be administered 1 to 7 days per week to a patient under therapy for treating CCM.

The composition according to the present invention can be administered according to a continuous schedule.

The present invention also relates to a method for preventing or treating cerebral cavernous malformations (CCM) by the administration of a pharmaceutical composition as defined above.

The present invention also relates to a method for preventing or treating cerebral cavernous malformations (CCM) by the administration 1 to 4 times daily of a pharmaceutical composition as defined above.

The present invention also relates to a method for preventing or treating cerebral cavernous malformations (CCM) by the daily administration 1 to 7 days per week of a pharmaceutical composition as defined above, the administration able to be carried out or not according to a continuous schedule.

The present invention is illustrated without limitation by the following examples.

### BRIEF DESCRIPTION OF THE FIGURES

- Figure 1 comprises graphs showing that targeting TRPV4 restores the proliferative defects of CCM2-depleted endothelial cells, and
- Figure 2 comprises graphs and images showing that targeting TRPV4 restores the permeability barrier of CCM2-depleted endothelial cells.
- Figure 3 comprises graphs showing that targeting TRPV4 restores ECM degradation by CCM2-depleted endothelial cells.

### EXAMPLE

### 1. Materials & Methods

### Cell culture and transfections:

Pooled Human Umbilical Vein Endothelial Cells (HUVEC) were obtained from Pelobiotech. Upon reception, HUVEC at P0 were expanded over 2 passages in 100µg/ml collagen 1 (from rat tail, BD) coated flasks in complete EGM-2 medium (Lonza) supplemented with 100 U/ml penicillin and 100 µg/ml streptomycin at 37°C in a 5% CO2 - 3% O2 humidified chamber. HUVEC at passage 3 were transfected twice at 24 h-intervals with 30 nM siRNA and Lipofectamine RNAi max (Life Technologies, ref. 13778-150) according to the manufacturer's instructions in OptiMEM at 37°C in a regular 5% CO2 in a humidified chamber. For double transfections, 30 nM of each siRNA duplexes (Dharmacon smartpool ON-TARGET plus Thermo Scientific) was used: Non-targeting siRNA #1 (ref. D-001810-01), CCM2 siRNA (ref. L-014728-01) and TRPV4 siRNA (ref. L-004195-00).

### Immunofluorescence staining:

Transfected HUVEC were seeded at 3x104 cells (sparsed, for Ki67 staining) or 1.5x106 cells (confluent, for VE-cadherin staining) in 24-well plates on coverslips coated with 10 µg/ml fibronectin (from human plasma, Sigma Aldrich) and incubated overnight (Ki67 experiment) or for 48h (VE-cadherin experiment) in complete EBM-2 medium in presence or absence of the calcium channels inhibitors dissolved in DMSO. For accurate comparison, DMSO was also added in the sample without inhibitor at the maximum concentration used. Cells were fixed with 4% PFA, permeabilized with 0.2% Triton X-100, and incubated with anti-Ki67 (AN9260 Millipore, 1/200) or anti-VE-cadherin (BV9 Millipore MABT129) antibodies. After rinsing, coverslips were incubated in Goat anti-Mouse IgG (H+L) highly cross-adsorbed secondary antibody, Alexa Fluor conjugated AF 488 (Invitrogen, 1/1000). The coverslips were mounted in Mowiol/DAPI solution and imaged on an epifluorescent Axiomager microscope (Zeiss) at 63X magnification. The percentage of Ki67 positive cells was counted manually out of more than 130 cells total.

### SA-B-Galactosidase staining:

Transfected HUVEC were seeded 48 hours after the second siRNA transfection at a density of 3x104 in 24-well plates coated with 10µg/mL fibronectin and incubated overnight in complete EBM-2-medium in presence or absence of the calcium channels inhibitors. For accurate comparison, DMSO was also added in the sample without inhibitor at the maximum concentration used. Senescence-Associated β-galactosidase activity was assessed using a SA-β-galactosidase staining kit according to the manufacturer's instructions (Cell Signaling). Positive cells were counted manually out of more than 130 cells total.

### xCELLigence proliferation assay:

Proliferation assay was performed using the xCELLigence Real-Time Cell Analysis (RTCA) DP instrument in combination with E-plate 16 (ACEA Biosciences) coated with 100µL of 100µg/mL collagen 1 (from rat tail) for 30 min at 37°C and washed 2 times with PBS 1X. 50 µL of complete EBM-2 medium was added and baseline without cells was made with RTCA software. 2.5x103 transfected HUVEC (4 wells per condition) were seeded 24h after the second round of transfection in 50µL of complete EBM-2-medium. Channels inhibitors or equivalent DMSO concentration were added immediately after seeding in 50µl, at 3 times the final desired concentration. Impedance measurements were recorded every 5 min during 24h. Impedance was normalized at 4 hours after seeding to eliminate the contribution of cell spreading and adhesion to the signal. Slope measurement was performed between 4h and 24h.

### Permeability assay:

The xCELLigence real-time cell analyzer (RTCA) system (Ozyme) was used to measure electrical impedance over time. Changes in impedance of confluent endothelial cells reflect changes in barrier function (Twiss et al., 2012). 4x104 transfected HUVEC were seeded at confluence in EGM-2 complete media in wells of E-plates 16 (at least 4 wells per condition) previously coated with 10ug/ml vitronectin and 50ug/ml collagen I. After 4h of adhesion, cells were starved in serum and cultured in basal EBM-2 containing 0.3% BSA in presence or absence of the calcium channels inhibitors for another 24 h. Cell index was normalized to the time of serum starvation to eliminate the signal due to cell spreading and adhering to the substrate.

### Gelation degradation assay:

Coverslips in 24 well-plates were coated with gelatin-Alexa488 dye as previously described (70). siCT and siCCM2 HUVEC were seeded 24h after the second siRNA transfection at 3x104/well in OptiMEM medium in presence or absence of the calcium channels inhibitors overnight at 37°C in a 5% CO2 incubator. Cells were fixed with 4% PFA, washed with PBS twice. The coverslips were mounted in Mowiol/DAPI solution and imaged on an epifluorescent Axiomager microscope (Zeiss) at 63X magnification. For a quantitative analysis of the degradative skill of siRNA transfected cells, 10 images per condition were randomly acquired and were converted to binary images using B&W thresholding on Imaged. The total area of the black zones corresponding to the total area of degradation of the fluorescent gelatin was measured.

### 3D-PEG invasion assays:

Poly-ethylene glycol (PEG) hydrogels were prepared on ice in EBM-2 complete media by combining an MMP-sensitive peptide modified PEG precursor (8-arm 40kDa,(71)) at 1.5% polymer concentration, 50 µM Lys-RGD peptide (Pepmic), and 1 µM sphingosine-1-phosphate (S1P, Sigma-Aldrich). The hydrogels were enzymatically crosslinked using a reconstituted and thrombin-activated Factor XIII (Fibrogammin, CSL Behring), prepared as previously described (72)) at 10% of total hydrogel volume. A 20 µL volume of the hydrogel suspension was pipetted into a modified imaging chamber (Secure-Seal^{™} hybridization sealing systems, ThermoFisher Scientific) attached to the bottom of a 24-well plate held vertically (62). The hydrogel was allowed to polymerize for 30 minutes in this orientation at room temperature prior to cell seeding. Depending on the study, a confluent cell monolayer composed of either 5X104 siRNA transfected GFP-HUVEC or a 1:1 ratio mixture of siRNA transfected GFP-HUVEC and naive RFP-HUVEC was allowed to adhere to the PEG meniscus at 37°C, 5% CO2 for 1 hour and then placed back horizontally with 1mL of EBM-2 complete media. Fixation with 4% PFA in DPBS was performed after 24 hours. Sprouts invading the PEG hydrogel were imaged using a Leica SP8 inverted confocal microscope with an HC PL APO 10x, 0.4 numerical aperture dry objective to obtain image stacks at 1024x1024 pixels with a 50 µm Z-stack at 1-1.5 µm Z-spacing. A Z-projection of each image was used to manually quantify invasion distances using the line measurement tool of Imaged. More than 100 sprouts were analyzed per technical replicate.

### Statistical tests:

Results were assessed by either performing a paired t-test for comparing 2 conditions or for more than 2 conditions by the Tukey's multiple comparison tests post-ANOVA to compare with control; a 0.5 alpha level was used for all comparisons. Prism software was used to conduct the statistical analysis of all data. P<0.05 was considered to be significant. *P<0.05, **P<0.05, ***P<0.005. n represents biological replicates.

### 2. Results

### Restoring effect of siTRPV4 and GSK2193874 (TRPV4 antagonist) on:

### ➢ The Senescence Associated with a Secretory Phenotype (SASP) of CCM2-depleted endothelial cells

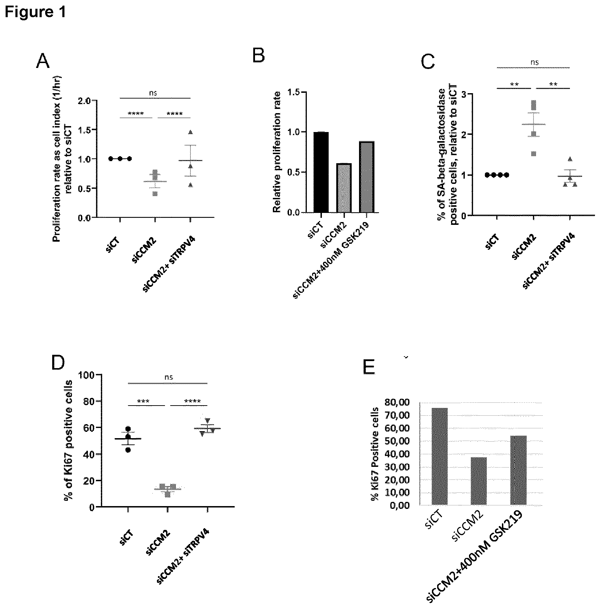

Referring to figure 1, upon CCM2 depletion, HUVECs undergo a reprogramming towards senescence (Vannier et al., Angiogenesis, 2021). Targeting TRPV4 by specific siRNAs (siTRPV4) blocks the senescence of the CCM2-depleted ECs (siCCM2) as shown by the restoration of the control (siCT) level of cells positive for Senescence-Associated beta-galactosidase activity (C) and restores their proliferative capacity as shown with the proliferative marker Ki67 immunostaining (D) and their proliferation rate as measured by RTCA xCelligence impedance assay over 24h (A). Similarly, a restoration of proliferative marker Ki67 (E) and proliferation rate (B) was observed with 400nM of GSK2193874 (named "GSK219" on the figures), a specific TRPV4 antagonist added to siCCM2 ECs.

### ➢ the permeability barrier of CCM2-depleted endothelial cells

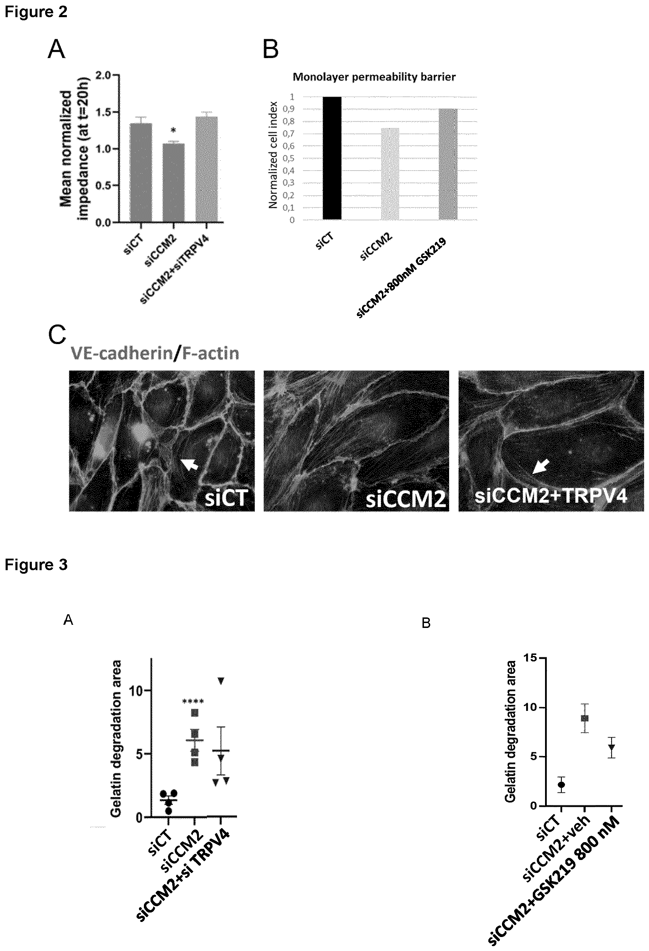

Referring to figure 2, CCM2 loss triggers the loss of endothelial junction permeability (Stockton et. al, J Exp Med, 2010) which is prior to ECM invasion and CCM lesion formation and leads to blood haemorrhage. Targeting TRPV4 by specific siRNAs restores barrier permeability as seen with impedance measurements on confluent cells using RTCA xCelligence (A). Similar restoration was observed with 800nM of GSK2193874 (B). By immunofluorescence staining, the cortical organization of actin was restored with siRNA treatment (arrow, C). Upon CCM2-depletion, actin stress fibers are formed and stretch the cells apart, weakening the junctions. Restoration by siTRPV4 allows the cortical support architecture for cell-cell junctions.

### ➢ the degradation of the ECM by CCM2-depleted endothelial cells

Referring to figure 3, upon CCM2 depletion, HUVECs acquire the capacity to degrade the extracellular matrix (ECM) as a way to invade it (Vannier et al., Angiogenesis, 2021). Targeting TRPV4 by specific siRNAs (A) or using 800 nM GSK2193874 (B) inhibits CCM2-depleted ECs capacity to degrade the ECM such as gelatin (denatured collagen).

## Claims

1. A pharmaceutical composition for use in the prevention or treatment of cerebral cavernous malformations (CCM) comprising a transient receptor potential vanilloid 4 (TRPV4) inhibitor.

2. The pharmaceutical composition for use in the prevention or treatment of CCM according to claim 1, wherein the TRPV4 inhibitor is selected among a TRPV4 antagonist and a TRPV4 RNA interference agent.

3. The pharmaceutical composition for use in the prevention or treatment of CCM according to claim 2, wherein TRPV4 RNA interference agent is selected from the group consisting of TRPV4 siRNA, TRPV4 shRNA, micro-RNA, TRPV4 aiRNA.

4. The pharmaceutical composition for use in the prevention or treatment of CCM according to claim 3, wherein TRPV4 RNA interference agent is TRPV4 siRNA.

5. The pharmaceutical composition for use in the prevention or treatment of CCM according to claim 2, wherein TRPV4 antagonist is selected from the group consisting of GSK2193874, GSK2798745, HC 067047, RN-1734, GSK3395879, GSK3491943 or GSK3527497, RN-9893, PF-05214030.

6. The pharmaceutical composition for use in the prevention or treatment of CCM according to claim 5, wherein TRPV4 antagonist is GSK2193874.
